# EUROPEAN PATENT APPLICATION

(11) **EP 1 482 030 A1**
(43) Date of publication of application: **01.12.2004**
(21) Application number: 03012106.5
(22) Date of filing: 28.05.2003
(51) Int. Cl.: C12N 5/06, C12N 5/08, A61K 35/36

(54) **Method for increasing the yield of proliferating primary keratinocytes**

(71) Applicant: Deutsches Krebsforschungszentrum Stiftung des öffentlichen Rechts, 69120 Heidelberg (DE); Boehringer Ingelheim International GmbH, 55216 Ingelheim am Rhein (DE)
(72) Inventor: Angel, Peter, 67127 Rödersheim (DE); Szabowski, Axel, 69118 Heidelberg (DE); Wagner, Erwin F., 1030 Wien (AT)
(74) Representative: Isenbruck, Günter, Dr.

(57) **Abstract**

Disclosed is a method for cultivating keratinocytes and increasing the yield of proliferating keratinocytes by using fibroblasts the JNK activity of which is eliminated or decreased, the use of said fibroblasts and keratinocytes in wound healing and a method for the identification of a JNK-regulated secretable factor.

## Description

The present invention refers to a novel method for increasing the yield of proliferating primary keratinocytes by using genetically modified fibroblasts as feeder layers. The disclosed method allows the maintenance of primary keratinocytes in a proliferating rather than in a terminally differentiated state. Such a system is particularly useful for skin transplantation and the therapeutic treatment of other diseases associated with the skin.

The skin is composed of the so-called epidermis, an external epithelial component, which is separated by a basement membrane from the so-called dermis, the underlying connective tissue component. The papillary layer which is the portion of the dermis adjacent to the epidermis consists of microvessels, fibroblasts, migrating leukocytes and collagen fibres and supplies essentially all of the nutrition to the epidermis which is devoid of blood vessels. The epidermis itself is primarily composed of keratinocytes which are arranged in stratified layers. The so-called stratum basale at the dermal-epidermal junction is a single layer of keratinocytes with a small number of interspersed melanocytes. The stratum basale is also known to the person skilled in the art as stratum germinativum since it is the site of the generation of new keratinocytes by cell proliferation.

The keratinocytes in the stratum basale can be divided in three subpopulations: stem cells which form so-called holoclones, transit-amplifying cells forming meroclones and committed cells which give rise to paraclones. Stem cells representing approximately 10% of the basal cell population can divide by mitosis to produce both daughter stem cells and cells that go on to differentiate (transit-amplifying cells). Transit-amplifying cells representing approximately 40% of the basal cell population undergo a limited number of divisions before they withdraw from the cell cycle, commit to terminal differentiation, detach from the basement membrane and begin to migrate towards the surface of the skin where they are sloughed off as dead, enucleated, and flattened cells called squames.

Adult, human epidermal proliferating keratinocytes can be maintained and propagated *in vitro* and applied for several applications including *in vitro* toxicity testing, for autografting and allografting to aid in wound healing, to investigate the feasibility of *ex vivo* gene therapy approaches and to study the basic biology of theses cells. In principle, a small but pure population of holoclone-generating cells would be all that is required for epidermal grafts. Skin autografts are produced by culturing keratinocytes to generate an epidermal sheet, and then transplanting this sheet to the affected site of the body. A common technique established 1975 by Rheinwald and Green is the growth and subcultivation of keratinocytes isolated from biopsies in order to propagate them *in vitro* on a so-called "feeder layer" of fibroblasts growth arrested by irradiation or mitomycin C of human or rodent origin (e.g. murine 3T3 fibroblasts). Fibroblasts usually secrete certain cytokines and other growth factors like e.g. keratinocyte growth factor (KGF) or Granulocyte Macrophage-Colony Stimulating Factor (GM-CSF) which are required for keratinocyte differentiation. The use of irradiated fibroblasts for *in vitro* culturing of keratinocytes prevents that the fibroblasts overgrow the keratinocytes to be propagated.

However, experimental conditions can cause depletion of the holoclone-generating compartment in cell cultures, similar to the partial depletion of the stem cell compartment occurring *in vitro* (e.g. during aging). Retention or depletion of true stem cells in a keratinocyte population *in vitro* is significantly affected by the composition of the culture medium, the nature of the carrier or substrate used for culturing and grafting, and could account for instances of graft failure in clinical practice. The long-term success of a skin graft thus depends on appropriate replenishment of stem cells.

Limiting for the therapeutic purposes described supra is the fact that proliferating keratinocytes can be maintained in culture only for approximately 15 to 20 population divisions before they enter terminal differentiation, resulting in an insufficient amount of keratinocytes available for therapeutic or other purposes. Thus, the problem underlying the present invention is the limited propagation of keratinocytes in culture due to a shift from proliferating into terminally differentiated cells during increasing number of cell divisions. A second problem to be solved by the present invention is an insufficient maintenance of a pure population of proliferating keratinocytes since with the presently available culture conditions the cell population is usually mixed with cells that have entered the differentiation stage.

According to the available state of the art, in the past the problem was tried to be solved by adding diverse growth factors or other proliferation-enhancing compounds to the culture medium. For example, Shipley et al. report in J. Cell. Physiol., 1989, Vol. 138, pages 511-518, that acidic as well as basic Fibroblast Growth Factor (aFGF; bFGF) stimulate the proliferation of keratinocytes cultured in serum-free medium. Furthermore, it has been observed that a distinct ratio between epidermal growth factor (EGF) versus calcium ions added to the culture medium determine the fate of whether keratinocytes rather proliferate of differentiate. Wille et al. describe in J. Cell. Physiol., 1984, Vol. 121, pages 31-44 that a combination of high EGF and low calcium promote proliferation whereas low EGF and high calcium shifts the balance towards differentiation. However, these techniques require the testing of many experimental conditions with various combinations of proliferation-enhancing factors in order to establish suitable parameters.

The international patent application WO-A 01/51619 discloses a genetically modified fibroblast cell exhibiting an altered activity of the transcription factor complex AP-1 (also known as Jun/Fos complex) due to the inactivation of one or more subunits (for example c-Jun) of said transcription factor complex. Using an *in vitro* skin equivalent model strong evidence was provided for a critical and specific function of c-Jun (and JunB) in mesenchymal-epithelial interaction in the skin by regulating the expression of interleukin-1 (IL-1)-induced keratinocyte growth factor (KGF) and GM-CSF in fibroblasts. These factors, in turn, adjust the balance between proliferation and differentiation of keratinocytes ensuring proper architecture of the epidermis. Keratinocytes which are grown using fibroblasts deficient in AP-1 (c-Jun or JunB subunit) as feeder layer show an altered proliferation and differentiation pattern as compared to keratinocytes which are co-cultivated together with wildtype fibroblasts, most likely due to the fact that an AP-1 regulated factor which is normally secreted by fibroblasts and required for keratinocyte proliferation/differentiation, like KGF (for proliferation) or GM-CSF (for differentiation), is missing. However, the system does not provide a method the application of which reversibly maintains a growing keratinocyte culture in a predominantly proliferating state, which can be shifted to differentiation without losing the general properties of the keratinocytes.

The applicant has now found that keratinocytes being co-cultivated together with fibroblasts that are deficient in the so-called c-Jun N-terminal kinase (JNK), also known to the skilled artisan as stress-activated protein kinase (SAPK), can be maintained as predominantly proliferating stem cells *in vitro* for numerous cell divisions. JNK/SAPK belongs to the so-called Mitogen-Activated Protein Kinase (MAP Kinase) superfamily and phosphorylates for example the transcription factor c-Jun described supra in the context of WO-A 01/51619. The effect can be reversed by adding a protein fraction of 10 to 30 kilo Dalton obtained from the medium of wildtype fibroblasts which leads to the keratinocytes undergoing terminal differentiation. The maintenance of proliferating keratinocytes being in co-culture with JNK-deficient fibroblasts is not due to possibly lacking c-Jun phosphorylation cell because with fibroblasts having normal JNK activity but bearing a c-Jun mutant with mutated phosphorylation sites the co-cultured keratinocytes enter differentiation. The decision for proliferation or differentiation thus relies on at least one novel compound which is secreted from wildtype fibroblasts and renders keratinocytes susceptible for differentiation-inducing signals (e.g. GM-CSF) but which is missing in JNK-deficient fibroblasts.

Therefore, a first object of the present invention refers to a method for cultivating keratinocytes, which method comprises the following steps:
(a) providing fibroblasts, the JNK activity of which is decreased or eliminated,
(b) providing epidermal keratinocytes
   and
(c) co-cultivating the fibroblasts from (a) together with the keratinocytes from (b).

The method of the present invention is suitable for increasing the yield of proliferating keratinocytes.

In the context of the present the activity of all JNK isoforms present in fibroblasts, as well as their respective splice variants can be affected. Preferably, the activity of p46 JNK1, and/or p54 JNK2 is decreased or eliminated.

The term "decrease or elimination" of the JNK activity refers to any method known to the person skilled in the art or compound that is capable of directly modulating the expression and/or the function of JNK, thus leading to the malfunction or down-regulation of JNK-dependent factors or targets. It is further contemplated within the scope of the present invention that the JNK activity is decreased or eliminated indirectly, namely by affecting the expression and/or the function of factors which in turn regulate the expression and/or the function of JNK. It is obvious to the person skilled in the art that a down-regulation or modification of JNK-regulating factors directly interferes with the function of JNK itself. Therefore, the hereinafter described mechanisms and molecules, respectively, which modulate the expression and/or the function of JNK may also be extrapolated and extended to JNK-regulating factors. Therefore, decrease and elimination of JNK activity contemplates also decrease and elimination of JNK-regulating factors and/or their activity. Examples of such JNK-regulating factors include small GTP-binding proteins such as Rac/Ccd42 and p21-activated kinase (PAK), involved in gathering external stimuli on the cell surface, MAP kinase kinase (MEK-4, -7) which phosphorylates JNK/SAPK or MEKK1-3 (MEK kinase).

The decrease or elimination can occur on the nucleic acid level by a peptide or a nucleic acid that regulates the transcription of the JNK gene by binding to up-stream and/or downstream regulatory sequences of the coding region of JNK. Such regulatory sequences are known to the person skilled in the art and include so-called promoter, operator, enhancer or silencer regions.

In a further embodiment of the present invention, the decrease or elimination on the nucleic acid level can occur by the use of nucleic acid molecules that hybridize to, and are therefore complementary to the coding sequence of the JNK gene. These nucleic acid molecules may encode or act as JNK gene antisense molecules useful, for example, in JNK gene regulation. The use of antisense molecules as inhibitors is a specific, genetically based therapeutic approach. The present invention provides the therapeutic and prophylactic use of nucleic acids of at least six nucleotides that are antisense to a gene or cDNA encoding one of the aforementioned JNKs.

Similarly, the JNK activity can be decreased / eliminated by a dsRNA molecule which is complementary to the JNK mRNA. Such molecules are also known in the art as small interfering RNA (siRNA). This technology to inhibit the expression of certain mRNAs is known to the person skilled in the art as RNA interference (RNAi). Preferably, the dsRNA molecules which are complementary to the mRNA of the cytokines of the present invention have a length between 10 and 30 base pairs, more preferably, they have a length between 19 and 25 base pairs. The JNK-specific siRNA may be delivered to the target fibroblast by any method known the one of skilled art. Applicable is, for instance, the delivery by using cationic liposome reagents.

Furthermore, the present invention encompasses the decrease or elimination of JNK activity by so-called ribozymes. Ribozymes are naturally occurring RNA fragments that can be designed as human therapeutics to recognize, bind and digest any mRNA sequence, in this case the JNK mRNA. Ribozymes are designed to target the JNK mRNA through complementary base pair hybridization. After binding to the target, the enzymatic activity of the ribozyme cleaves the JNK mRNA thus preventing its translation into protein. The JNK mRNA ribozymes can be chemically synthesized to selectively inhibit the JNK production.

In a further embodiment of the present invention the translation of the JNK gene can be reduced or eliminated by binding of an RNA-binding protein to one or more operator sequences in the 5'-UTR of the JNK mRNA transcript. The bound RNA-binding protein interferes with translation, likely by preventing ribosome assembly or blocking the movement of the ribosome along the transcript from 5' to 3'. Such RNA-binding proteins may be multimeric, e.g. dimers of a particular RNA-binding protein.

In the most preferred embodiment of the present invention, the purposeful decrease or elimination of the JNK activity is achieved by the generation of a transgenic "knock-out" animal lacking the *jnk* gene (refers to *jnk*1 and *jnk*2) on both alleles and the isolation of fibroblasts from such transgenic "knock-out" animals (*jnk* -/- fibroblasts). The generation of a "knock-out" animal, preferably a mammal, most preferably a mouse, is basically done following the method developed in the late eighties by Mario Capecchi, which is well-known to the person skilled in the art. In brief, the method includes the generation of a gene targeting vector useful for homologous recombination, the isolation of murine embryonic stem cells from the blastocyste, the introduction of the gene targeting vector into said stem cells allowing for the insertion into the desired site within the genome, the introduction of the genetically modified stem cell into another blastocyste, implantation of said blastocyste into a pseudo-pregnant mouse to obtain a chimerical animal, and, eventually, the pairing of such chimeras with wildtype mice for the generation of homozygous jnk -/- organisms.

If the decrease/elimination should occur on the protein level, the present invention encompasses antibodies or fragments thereof capable of specifically recognizing one or more epitopes of the JNK gene products. Such antibodies may include, but are not limited to, polyclonal antibodies, monoclonal antibodies (mAbs), humanized or chimeric antibodies, single-chain antibodies, Fab fragments, F(ab')2 fragments, Fv fragments, fragments produced by a Fab expression library, anti-idiotypic (anti-Id) antibodies, and epitope-binding fragments of any of the above.

Furthermore, the purposeful decrease/elimination on the protein level can be performed by employing compounds which impede the phosphorylation of JNK and, as a consequence, indirectly affect its activity. Included for these purposes are so-called small molecule inhibitors, either synthetic or naturally occurring inhibitors. Among the naturally occurring compounds which interfere with JNK phosphorylation / activity so-called phosphatases, de-phosphorylating enzymes, which can be overexpressed in the respective fibroblasts, are included in the present invention. Known phosphatases specific for JNK are MKP-1 (CL100), M3/M6 and MKP-2. Furthermore, overexpression of individual phosphatases like PP2A, which dephosphorylates Serin and Threonin residues, or PTP1, which dephosphorylates tyrosine residues can be employed.

The method for increasing the yield of proliferating keratinocytes can be performed by a two-dimensional feeder-layer co-culture system, either in a conventional or in a so-called trans-well fashion, or by employing a so-called three-dimensional, organotypical co-culture system leading to a multi-layered, ordered epithelium of keratinocytes. Preferably, the method of the present invention is performed by a conventional or trans-well two-dimensional co-culture system. The establishment of said co-culture system is substantially described in WO-A 01/51616, the content of which is incorporated herein by reference.

The method of the present invention can be applied to all epithelial cells whenever an *in vitro* enrichment of the respective proliferating stem cell is desired. Included are cells derived from surface epithelia such as mucosa, blood vessel endothelium, one-layer plate or prismatic epithelia (e.g. gut mucosa, uterus mucosa), multi-layer epithelia (cornified, e.g. skin, cornea; or uncornified), transition epithelia (e.g. ureter, bladder), gland epithelia, sense epithelia and myoepithelia. Most preferably, the method is applied to epithelial cells derived from skin.

In this respect, the method of the present invention, in particular the JNK-deficient fibroblasts can be used as a therapeutical tool for wound healing. Wounds can occur at any of the above described organs and tissue. The JNK-deficient fibroblasts of the present invention can be employed for wound healing and tissue regeneration in any of these. As an example, wound healing and tissue regeneration of the skin is considered. Wounds of the skin to be treated by using the JNK-deficient cells of the present invention encompass burn wounds as well as excision wounds. The burn is one of the most common types of skin damage to require medical attention. There exist four major types of burn: thermal, chemical, electrical and radiation (e.g. sunburn), all of which can be treated by the present method. Severe burn wounds require an immediate removal / excision of the burned and an instant replacement by new cells/tissue. Thus, the method and the JNK-deficient cells of the present invention provide an efficient tool to rapidly propagate keratinocytes in culture in order use them for transplantation and regeneration.

To fulfil the required criteria for the therapeutic purposes described supra, i.e. the rapid propagation of proliferating keratinocytes, it is desired that the method of the present invention by co-culturing JNK-deficient fibroblasts increases the yield of proliferating keratinocytes to obtain a substantially pure population of keratinocytes, which is another object of the present invention. Consequently, the present invention further refers to the use of a cultured keratinocyte population containing at least 80% proliferating stem cells for wound healing. Preferably, the cultured keratinocyte population to be used for wound healing contains at least 90% proliferating stem cells, more preferably it contains at least 95% proliferating stem cells, and most preferred it contains at least 99.9% proliferating stem cells.

The proliferation state of the keratinocytes population can be proven by the detection of proliferation-specific marker proteins which are well-known to the skilled artisan. Examples for proliferation-specific marker proteins include the nuclear protein Ki-67, also known as MIB-1, keratin K19 and β1-integrin. Preferably, the proliferation state of stem cell co-cultivated with JNK activity lacking fibroblasts is proven by the detection of Ki-67 or by the lack of differentiation markers described infra.

In a further embodiment, the present invention refers to a method to identify a compound which modulates (enhances or inhibits) the JNK-dependent proliferation and/or differentiation of keratinocytes, the method comprising the following steps:
(a) contacting the test compound with fibroblasts in co-culture with primary keratinocytes, wherein said fibroblasts have a decreased or eliminated JNK activity,
(b) contacting said test compound with fibroblasts in co-culture with primary keratinocytes, wherein said fibroblasts have a regular JNK activity,
(c) detecting the expression of proliferation- or differentiation-specific marker proteins in the keratinocytes culture of (a) and of (b)
   and
(d) comparing the expression of proliferation- or differentiation-specific marker proteins in the keratinocytes culture of (a) and of (b).

Proliferation- or differentiation-specific marker proteins are well-known in the art and are described within the present invention.

The method to identify a compound which modulates (enhances or inhibits) the JNK-dependent proliferation and/or differentiation of keratinocytes can be performed by a two-dimensional feeder-layer co-culture system, either in a conventional or in a so-called trans-well fashion, or by employing a so-called three-dimensional, organotypical co-culture system leading to a multi-layered, ordered epithelium of keratinocytes. Preferably, the method of the present invention is performed by a conventional or trans-well two-dimensional co-culture system.

Another object of the present invention refers to a method for the identification of a secretable JNK-regulated factor, wherein the presence or absence of said JNK-regulated factor or the presence or absence of its activity determines the proliferation and/or differentiation state of a co-cultured epithelial stem cell, e.g. a keratinocyte stem cell or transit-amplifying cell. The method comprises the following steps:
(a) providing an in vitro culture of fibroblasts with regular JNK activity,
(b) providing an in vitro culture of fibroblasts with decreased or eliminated JNK activity,
(c) removing the cell culture supernatant I from the culture of (a) and the cell culture supernatant II from the culture of (b),
(d) screening said supernatants I und II for their ability to render proliferating keratinocytes susceptible for entry into differentiation,
(e) subjecting the cell culture supernatants I and II obtained in (c) to various purification and/or filtration and/or fractionation steps,
   and
(f) comparing the protein pattern of cell culture supernatant I with the protein pattern of cell culture supernatant II to identify one or more factor being present in cell culture supernatant I, but not in II, or to identify one or more factor being present in cell culture supernatant II, but not in I.

Thus, another object of the present invention refers to an isolated and purified differentiation-promoting factor, which is not present in jnk -/- fibroblasts, wherein the presence and/or activity of said factor depends on the activity of JNK and wherein the activity of said factor mediates the susceptibility of proliferating keratinocytes and transit-amplifying cells to enter terminal differentiation. The differentiation-promoting factor according to the present invention is a molecule or a factor which can be represented by a definable complex of molecules, e.g. one or more proteins, and which possesses a biological activity being apparent by the detection of differentiation-specific marker proteins. Typical differentiation-specific marker proteins which should be detectable in differentiated keratinocytes upon the application of the differentiation-promoting factor of the present invention or a fraction containing it include keratin K1/K10, transglutaminase, involucrin, filaggrin, and loricrin. Preferably, the differentiation is measured by the detection of K1/K10.

The differentiation-promoting factor of the present invention is further characterized in that it is contained in a fraction obtained by size exclusion filtration of cell culture supernatant I from a culture of fibroblasts with regular JNK activity, wherein said fraction is centred around an apparent molecular weight between 10 and 30 kilo Dalton. The differentiation-promoting factor can be any secretable factor, like e.g. a growth factor or a component of a growth factor complex.

Mammalian JNKs are implicated in responses to cellular stress, inflammation and apoptosis. They are activated by lipopolysaccharides, interleukin-1 (IL-1), tumor necrosis factor alpha (TNF-α), ionizing or ultraviolet radiation, translation inhibitors cycloheximide and anisomycin, tumor promoters, heat shock or hyper osmotic shock. Furthermore, the induction of the breast cancer susceptibility gene BRCA1 triggers apoptosis through activation of JNK. In line with the prior art, the inventors have furthermore found out, that the activity of the differentiation-promoting factor is enhanced upon the addition of IL-1 to the cell culture.

Thus, the differentiation-promoting factor of the present invention can be used as a diagnostic sensor for any of the above described pathological conditions whenever the expression and/or the activity of the differentiation-promoting factor is altered as a result of a disease-related activation or inactivation of JNK as compared to the non-pathological situation.

Consequently, it is obvious to the skilled artisan that a down-regulation of the differentiation-promoting factor, for example by the purposeful decrease and elimination of the JNK activity according to any method described supra, can be employed in the treatment of any disease accompanied or caused by elevated levels of the differentiation-promoting factor of the present invention like cellular stress, inflammation, apoptosis or abnormal cell growth. On the other hand, an up-regulation or addition of the differentiation-promoting factor can be desired in highly proliferating tumor cells to stop the tumor cells from proliferation/growth and drive them into differentiation.

On the contrary, since the absence of the differentiation-promoting factor in JNK-deficient fibroblasts maintains said fibroblasts in a proliferating state, an up-regulation or ectopic application of the differentiation-promoting factor can be helpful in the treatment of diseases accompanied with extensive proliferation due to a decreased or eliminated level of the differentiation-promoting factor of the present invention, e.g. hyperproliferative skin diseases like psoriasis.

### BRIEF DESCRIPTION OF THE DRAWING

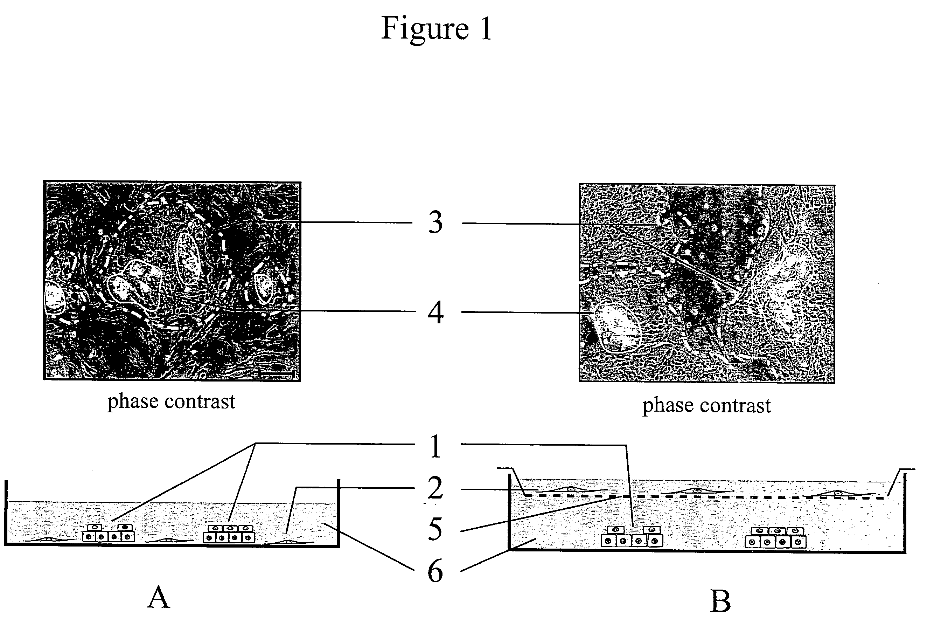

### Figure 1: Schematic representation of two-dimensional feeder-layer co-cultures

In conventional feeder-layer cocultures (A) as well as in the "transwell" (B) system primary human keratinocytes (1) are cultivated submerged in culture medium (6) in the presence of growth arrested murine fibroblasts (2). Under both conditions, keratinocytes proliferate forming the characteristic keratinocytes islands (3) (the borders of the islands are marked by dotted lines in the phase contrast microscopy) with areas of differentiated cells (4). Stratification of the keratinocytes within these islands indicates the ongoing terminal differentiation process of these cells as monitored by phase contrast microscopy. In the conventional two-dimensional feeder-layer system, the keratinocytes as well as the fibroblast feeder-layer are seeded side by side on the petri dish (A). In this experimental set-up both cell types can interact via paracrine signalling as well as direct cell-cell contacts. In contrast, in the "trans-well" feeder-layer coculture system (B) fibroblasts are seeded on a filter insert (5) separating them physically from keratinocytes. The pore size of these inserts allows the penetration of soluble factors, such as cytokines, but prevents direct cell-cell contacts of fibroblasts and keratinocytes

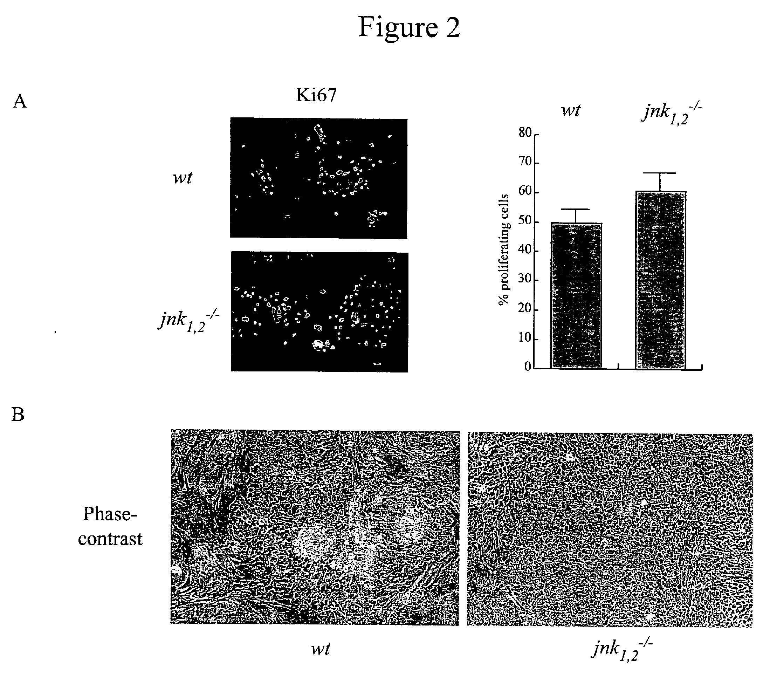

### Figure 2: Keratinocytes in co-culture with JNK-deficient fibroblasts to not enter terminal differentiation program

Panel A shows an immunofluorescence image of keratinocytes co-cultured with wildtype (wt) or JNK-deficient (jnk_{1,2}-/-) using an antiserum against the proliferation-associated protein Ki-67. The calculated percentage of proliferating cells is depicted in the diagram. Panel B shows the respective phase contrast image.

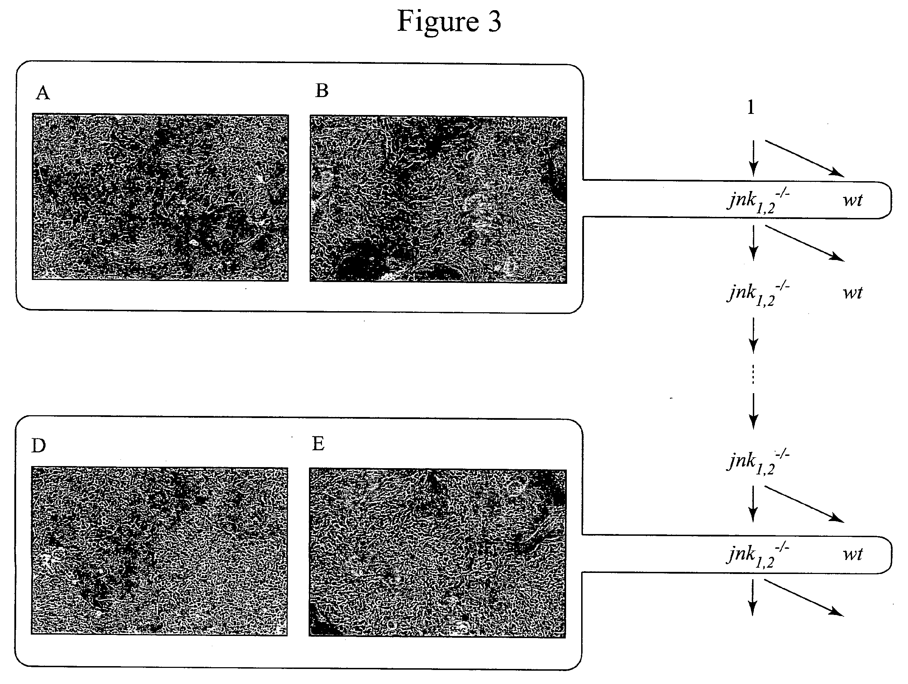

### Figure 3: Primary human keratinocytes can be passaged on a jnk1,2 -/- feeder-layer without alterations of their general properties

The upper panel shows primary human keratinocytes (1) in feeder-layer cocultures with JNK-deficient (jnk_{1,2}-/-) fibroblasts (A) or wildtype (wt) fibroblasts (B) in the second cell passage (C). The lower panel shows primary human keratinocytes (1) in feeder-layer cocultures with JNK-deficient (jnk_{1,2}-/-) fibroblasts (D) or wildtype (wt) fibroblasts (E) in the ninth cell passage (F).

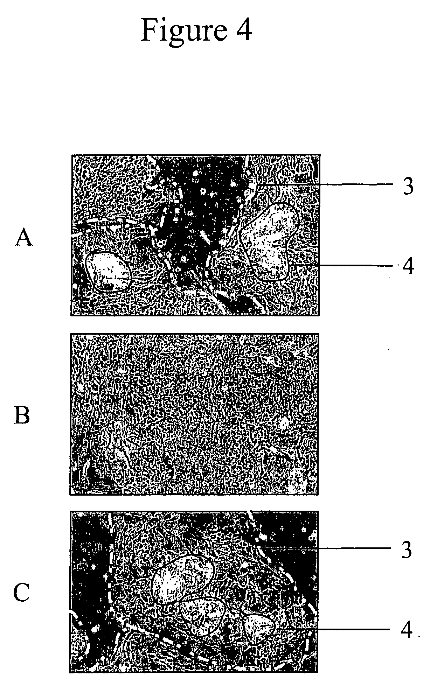

### Figure 4: Entry into the terminal differentiation program can be induced by a soluble 10-30 kDa factor

Panel A shows primary human keratinocytes in coculture with wildtype fibroblasts; Panel B shows primary human keratinocytes in coculture with JNK-deficient fibroblasts, and Panel Panel C shows primary human keratinocytes in coculture with JNK-deficient fibroblasts after the addition of supernatant from wildtype fibroblasts.

The invention is further illustrated by the following examples:

### EXAMPLES

### Example 1: Isolation and cultivation of primary human keratinocytes and fibroblasts

Epidermal keratinocytes and dermal fibroblasts were isolated from human skin biopsies. Keratinocytes were cultivated as feeder-layer co-cultures or trans-well feeder-layer co-cultures in FAD medium containing DMEM:Hams F12 3:1 with 0.1% glucose, 1mM L-glutamin, pH 7.3; with 100 U/ml penicillin, 10 µg/ml streptomycin, 5 µg/ml insulin, 1 ng/ml hEGF; 10⁻¹⁰ M choleratoxin, 24 ng/ml adenine (all compounds from SIGMA); and 5% FCS (1x10⁴/cm² feeder cells and 1.1x10⁴/cm² keratinocytes). For conventional feeder-layer co-cultures, uncoated cell culture dishes (Falcon; BD Biosciences) and for trans-well feeder-layer co-cultures, collagen type I - coated cell culture dishes (BD Biocoat; BD Biosciences) in combination with cell culture inserts (0.4 µm pore size; BD Biosciences) were used. Human fibroblasts were propagated in DMEM, 10% FCS. For the generation of feeder-layer fibroblasts, human fibroblasts were irradiated with 70 Gray (Gy) and murine fibroblasts with 20 Gy.

### Example 2: Fixation of cultures

The medium of monolayer cultures from adherent cells was withdrawn and the cells were fixed on glass slides in 80% methanol for 5 min and in 100% acetone for 3 min. The slides can be subsequently dried and stored at -40°C.

### Example 3: Keratinocytes in co-culture with JNK-deficient fibroblasts to not enter terminal differentiation program

Primary human keratinocytes in cocultures with JNK_{1,2}-deficient fibroblasts (see Figure 2) exhibit a slightly increased percentage of proliferating cells compared to cultures with wild type fibroblasts, as calculated in accordance with by immunofluorescence staining of the proliferation-associated protein Ki67 (A). Under these conditions, even after eight days in culture stratified layers are hardly detectable in cocultures with JNK_{1,2}-deficient fibroblasts as documented by phase contrast microscopy (B), indicating that the entry in the terminal differentiation program is not delayed but rather absent.

### Example 4: Primary human keratinocytes can be passaged on a jnk1,2 -/- feeder-layer without alterations of their general properties (Figure 3)

Primary human keratinocytes in feeder-layer cocultures with JNK-deficient fibroblast, see Figure 3, (A) or wild type fibroblast (B) in passage two (C). Normally, primary human keratinocytes (1) can only be further cultivated up to passage number three to four. This is due to the fact, that under these conditions the balance of proliferation and differentiation of keratinocytes is progressively shifted towards differentiation. Therefore, the percentage of proliferating cells gets reduced, whereas the number of stratifying areas becomes increased. In contrast, under the same conditions primary human keratinocytes grown in cocultures with JNK-deficient fibroblast (A) can be passaged and expanded far beyond this point. Even after nine passages (F) the appearance of differentiated keratinocytes is a rare event (D). If these high-passage primary human keratinocytes are seeded on a wild type fibroblast feeder-layer (E), they behave perfectly normal in terms of morphology, proliferation and the ability to differentiate. These data show that the general properties of the primary keratinocytes are obviously not altered, neither by factors derived from the JNK-deficient feeder-layer nor from the extended passaging and cultivation time.

### Example 5: Entry into the terminal differentiation program can be induced by a soluble 1.0-30 kDa factor (see Figure 4)

Using the trans-well coculture system (Figure 4), which prevents direct cell-cell contacts of the different cell types primary human keratinocytes cocultured with JNK-deficient fibroblasts initiate the terminal differentiation program with only very low efficiency (B), as compared to cocultures with wild type fibroblasts (A). These data rule out the possibility that the block of differentiation is due to alterations in direct cell-cell contacts between JNK-deficient fibroblasts and keratinocytes but is rather mediated by one or more fibroblast derived soluble factors, which are absent or expressed at insufficient levels in JNK-deficient cells. The ability of primary human keratinocytes in coculture with JNK-deficient fibroblasts to enter terminal differentiation can be restored by addition of supernatant of wildtype fibroblasts (C), which was collected by a 48 hrs culture period. Fractionation of this conditioned medium by size filtration revealed that the biologically active component has a molecular weight in the range from 10 to 30 kDa. Addition of this 10 to 30 kDa fraction of the conditioned medium normalises island size of growing keratinocytes (3) and induces terminal differentiation, as indicated by the reappearance of stratified keratinocytes (4). Vice versa, addition of a 10-30 kDa fraction from JNK-deficient fibroblast conditioned medium to cocultures with wild type fibroblasts does neither influences keratinocytes proliferation nor differentiation, excluding the existence of soluble factors exclusively expressed in JNK-deficient cells, which dominantly inhibit differentiation of keratinocytes.

## Claims

1. A method for cultivating keratinocytes, the method comprising the following steps
(a) providing fibroblasts, the JNK activity of which is purposefully decreased or eliminated,
(b) providing epidermal keratinocytes
and
(c) co-cultivating the fibroblasts from (a) together with the keratinocytes from (b).

2. The method according to claim 1, wherein JNK is JNK1 and/or JNK2.

3. The method according to claim 1 or 2, wherein the JNK activity is decreased or eliminated on the nucleic acid level.

4. The method according to claim 3, wherein the JNK activity is decreased or eliminated by the generation of a transgenic animal lacking the jnk1 and/or jnk2 gene.

5. The method according to claim 1 or 2, wherein the JNK activity is decreased or eliminated on the protein level.

6. The use of a fibroblast, the JNK activity of which is purposefully decreased or eliminated, for the manufacture of a medicament which can be applied in wound healing.

7. A cultured keratinocyte population obtainable by the method according to any of the claims 1 to 5, wherein the keratinocyte population contains at least 80% proliferating stem cells.

8. A cultured keratinocyte population according to claim 7, wherein the keratinocyte population contains at least 90% proliferating stem cells.

9. A cultured keratinocyte population according to claim 7 or 8, wherein the keratinocyte population contains at least 99.5% proliferating stem cells.

10. The use of a cultured keratinocytes population according to any of the claims 7 to 9 for the manufacture of a medicament which can be applied in wound healing.

11. A method to identify a component which modulates the JNK-dependent proliferation and/or differentiation of keratinocytes, the method comprising the following steps:
(a) contacting the test compound with fibroblasts in co-culture with primary keratinocytes, wherein said fibroblasts have a decreased or eliminated JNK activity,
(b) contacting said test compound with fibroblasts in co-culture with primary keratinocytes, wherein said fibroblasts have a regular JNK activity,
(c) detecting the expression of proliferation- or differentiation-specific marker proteins in the keratinocyte culture of (a) and (b),
and
(d) comparing the expression of proliferation- or differentiation-specific marker proteins in the keratinocyte culture of (a) and (b).

12. A method for the identification of a secretable JNK-regulated factor or its activity, wherein the presence or absence of said factor or the presence or absence of its activity determines the proliferation and/or differentiation state of a epithelial stem cell, the method comprising:
(a) providing a culture of fibroblasts with regular JNK activity,
(b) providing a culture of fibroblasts with decreased or eliminated JNK activity,
(c) removing the cell culture supernatant I from the culture of (a) and the cell culture supernatant II from the culture of (b),
(d) screening said supernatants I and II for their ability to render proliferating keratinocytes susceptible for entry into differentiation,
(e) subjecting the cell culture supernatants I and II obtained in (c) to various purification and/or filtration and/or fractionation steps,
and
(f) comparing the protein pattern of cell culture supernatant I with the protein pattern of cell culture supernatant II and identifying one or more factors being present in cell culture supernatant I, but not in II, or being present in cell culture supernatant II, but not in I.

13. A secretable JNK-regulated factor identified by the method according to claim 12.
